# EUROPEAN PATENT SPECIFICATION

(11) **EP 2 762 101 B1**
(45) Date of publication and mention of the grant of the patent: **04.08.2021**
(21) Application number: 14153210.1
(22) Date of filing: 30.01.2014
(51) Int. Cl.: A61B 18/14

(54) **Electrosurgical instrument**
Elektrochirurgisches Instrument
Instrument électro-chirurgical

(30) Priority: 05.02.2013 US 201361760941 P; 29.10.2013 US 201314065644
(43) Date of publication of application: 06.08.2014
(73) Proprietor: Covidien LP, Mansfield, MA 02048 (US)
(72) Inventor: Reschke, Arlen J., Longmont, CO 80501 (US)
(74) Representative: Soames, Candida Jane

(56) References cited:
- EP-A1- 2 425 791
- US-A- 5 658 281
- US-A1- 2008 275 445
- US-A1- 2012 310 233
- None

## Description

### BACKGROUND

### Technical Field

The present disclosure relates to an electrosurgical instrument and, more particularly, to an electrosurgical instrument including jaw members including a first electrode configuration and one or more second electrode configurations.

### Description of Related Art

Electrosurgical forceps are well known in the medical arts. For example, an electrosurgical endoscopic forceps is utilized in surgical procedures, e.g., laparoscopic surgical procedure, where access to tissue is accomplished through a cannula or other suitable device positioned in an opening on a patient. The endoscopic forceps, typically, includes a housing, a shaft, and an end effector assembly attached to a distal end of the shaft. The end effector includes jaw members that operably communicate with one another to grasp tissue. The jaw members may be configured to function in bipolar or monopolar energy deliver platforms. In a bipolar energy delivery platform, each jaw member includes an electrode configuration, and it is essential that tissue be in firm contact with both electrodes at one time. Unfortunately, a surgeon sometimes cannot manipulate the jaw members around tissue as a result of the relatively limited space within a body cavity. As can be appreciated, this may create an additional distraction (e.g., surgeon may become preoccupied with electrode orientation) for surgeons who are typically focused on the patient and tissue treatment.

EP2425791A describes an electrosurgical forceps according to the pre-characterizing portion of claim 1. US2008/275445A describes an expandable device for gastrointestinal tract ablation, which may have interlaced ablation electrodes. US2012/310233A describes expandable devices for electrosurgical treatment of an airway.

### SUMMARY

In view of the foregoing, an electrosurgical instrument including jaw members including a first electrode configuration and one or more second electrode configurations may prove useful in the surgical arena.

Aspects of the present disclosure are described in detail with reference to the drawing figures wherein like reference numerals identify similar or identical elements. As used herein, the term "distal" refers to the portion that is being described which is further from a user, while the term "proximal" refers to the portion that is being described which is closer to a user.

The present invention provides an electrosurgical forceps as defined in claim 1. The electrosurgical forceps includes a housing with a shaft that extends distally therefrom. An end effector assembly is operably coupled to a distal end of the shaft and includes a pair of first and second jaw members. One (or both) of the first and second jaw members is movable from an open configuration for positioning tissue therebetween, to a clamping configuration for grasping tissue therebetween. Each of the first and second jaw members includes a first electrode thereon for electrosurgically treating tissue. And, second and third electrodes are disposed on one of the first and second jaw members and arranged in an interlaced configuration relative to one another and separated by an insulator, the second and third electrodes configured to function in a bipolar configuration. The interlaced configuration is a T-shaped pattern.

The second and third electrodes may extend outwardly from one or both sides of the first or second jaw member. The second and third electrodes may be provided on the first or second jaw member via a photo etching process, conductive ink deposition process, laser deposition process, and a stamping process. The second and third electrodes may be configured to be active when the first electrodes on the first and second jaw members are active. Alternatively, the second and third electrodes may be configured to be inactive when the first electrodes on the first and second jaw members are active and is active when the first electrodes on the first and second jaw members are inactive.

The second and third electrodes may be provided on both of the first and second jaw members. The second and third electrodes may extend outwardly from either a left or right side of the first or second jaw member.

Suitably, the second and third electrodes are isolated from the first electrodes.

Suitably, the second and third electrodes are disposed on an insulator.

Suitably, the second and third electrodes are disposed on a flange.

Suitably, the second and third electrodes are disposed on a side relative to the jaw members grasping tissue therebetween.

Suitably, when tissue is grasped between the jaw members, the second and third electrodes may be brought into contact with tissue by rolling or tilting the jaw members.

Suitably, the second and third electrodes each comprise a spine portion and teeth portions extending therefrom, wherein the teeth of the second electrode interlace with the teeth of the third electrode.

Suitably, the second and third electrodes each comprise an extending (optionally longitudinally) portion and T-shaped portions projecting therefrom, the projecting portions of the second electrode interlacing with projecting portions of the third electrode.

Suitably, the second and third electrodes are disposed on a curved portion of the jaw member, curved in a cross-section perpendicular to a longitudinal axis of the end effector.

### BRIEF DESCRIPTION OF THE DRAWING

Various embodiments of the present disclosure are described herein below with references to the drawings, wherein:
Fig. 1 is a perspective view of an endoscopic electrosurgical forceps including an end effector according to an embodiment of the present disclosure;
Fig. 2 is a schematic, perspective view of a bottom jaw member of the end effector depicted in Fig. 1;
Fig. 3 is an enlarged view of the indicated area of detail of Fig, 2; and
Fig 4 is a side view of the jaw member depicted in Fig. 2 with a second electrode configuration provided thereon contacting tissue.

### DETAILED DESCRIPTION

Detailed embodiments of the present disclosure are disclosed herein; however, the disclosed embodiments are merely examples of the disclosure, which may be embodied in various forms. Therefore, specific structural and functional details disclosed herein are not to be interpreted as limiting, but merely as a basis for the claims and as a representative basis for teaching one skilled in the art to variously employ the present disclosure in virtually any appropriately detailed structure.

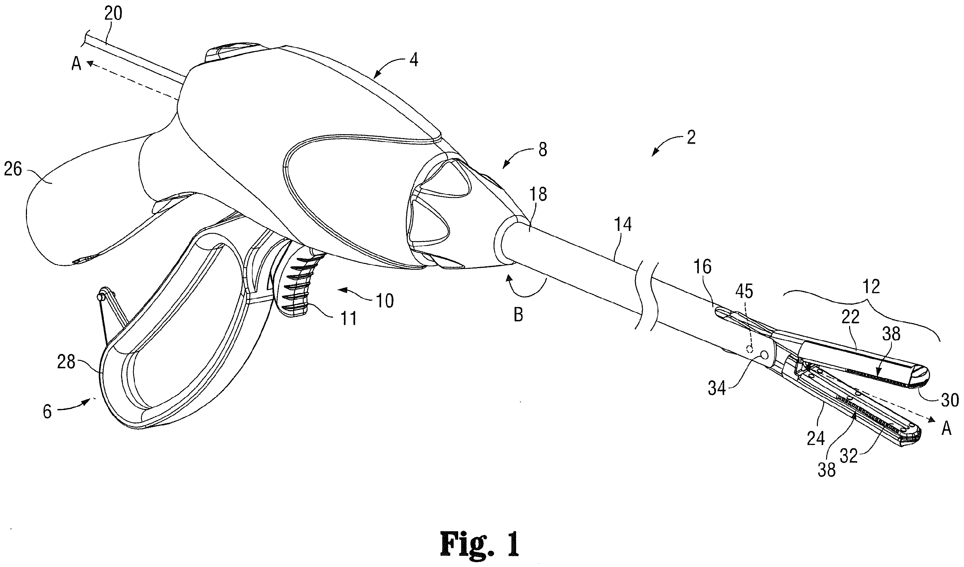

Turning now to Fig. 1, an electrosurgical endoscopic forceps 2 is illustrated. Forceps 2 includes a housing 4, a handle assembly 6, a rotating assembly 8, a trigger assembly 10 and an end effector assembly 12. Forceps 2 includes a shaft 14 that extends from housing 4 and has a longitudinal axis "A-A" defined therethrough. A distal end 16 of shaft 14 is configured to mechanically engage end effector assembly 12 and a proximal end 18 is configured to mechanically engage housing 4. Forceps 2 also includes an electrosurgical cable 20 that connects forceps 2 to a generator (not shown) or other suitable power source. Forceps 2 may alternatively be configured as a battery-powered instrument. Cable 20 includes a wire (or wires) (not explicitly shown) extending therethrough that has sufficient length to extend through shaft 14 in order to provide one or more suitable types of energy to one or both of a pair of jaw members 22 and 24 of end effector assembly 12. The generator may be configured to provide electrosurgical energy (e.g., RF, microwave), thermal energy, or ultrasonic energyto jaw members 22 and 24. In the illustrated embodiment, the generator is configured to provide RF energy to jaw members 22 and 24.

Rotating assembly 8 is rotatable in either direction about longitudinal axis "A-A" to rotate end effector 12 about longitudinal axis "A-A" (Fig. 1). Housing 4 houses the internal working components of forceps 2, such as a drive assembly (not explicitly shown), working components of handle assembly 6, electrical raceways associated with cable 20, and other working components therein.

Handle assembly 6 includes a fixed handle 26 and a moveable handle 28. Fixed handle 26 is integrally associated with housing 4 and movable handle 28 is moveable relative to fixed handle 26. Moveable handle 28 connects to the drive assembly such that, together, movable handle 28 and the drive assembly mechanically cooperate to impart movement of jaw members 22 and 24 between a spaced-apart position and an approximated position to grasp tissue disposed between electrodes 30 and 32 of jaw members 22, 24, respectively. As shown in Fig. 1, moveable handle 28 is initially spaced-apart from fixed handle 26 and, correspondingly, jaw members 22, 24 are in the spaced-apart position (Fig. 1). Moveable handle 28 is depressible from this initial position (Fig. 1) to a depressed position (not explicitly shown) corresponding to the approximated position of jaw members 22, 24.

Other methods for opening and closing the jaw members 22, 24 may be utilized. For example, any suitable number of linkage devices, gears, vacuum tubes, actuators and the like may be utilized alone or in combination with the movable handle 24 and/or drive assembly to impart movement of the jaw members 22, 24 from the spaced-apart position to the approximated position.

Continuing with reference to Fig. 1, end effector assembly 12 is designed as a bilateral assembly, i.e., where both jaw member 22 and jaw member 24 are moveable about pivot pin 34 relative to one another and to shaft 14. End effector assembly 12, however, may alternatively be configured as a unilateral assembly, i.e., where jaw member 24 is fixed relative to shaft 14 and jaw member 22 is moveable about pivot 34 relative to shaft 14 and fixed jaw member 24.

In the illustrated embodiment, jaw members 22, 24 are configured to function in bipolar mode of operation. Accordingly, each of jaw members 22, 24 includes a respective first electrode configuration including electrodes 30, 32, which serve as active and return electrodes, Alternatively, jaw members 22, 24 may be configured to function in a monopolar mode of operation in which case a return pad (not explicitly shown) may be utilized as a return electrode for providing a return path back to the generator for current. Electrodes 30, 32 are in operable communication with the generator via one or more leads (not explicitly shown) of cable 20 and are configured to provide electrosurgical energy to tissue grasped between jaw members 22, 24 (Fig. 1). A knife slot 53 may be defined through one or both of electrodes 30, 32. For illustrative purposes, only electrode 32 is shown with knife slot 53.

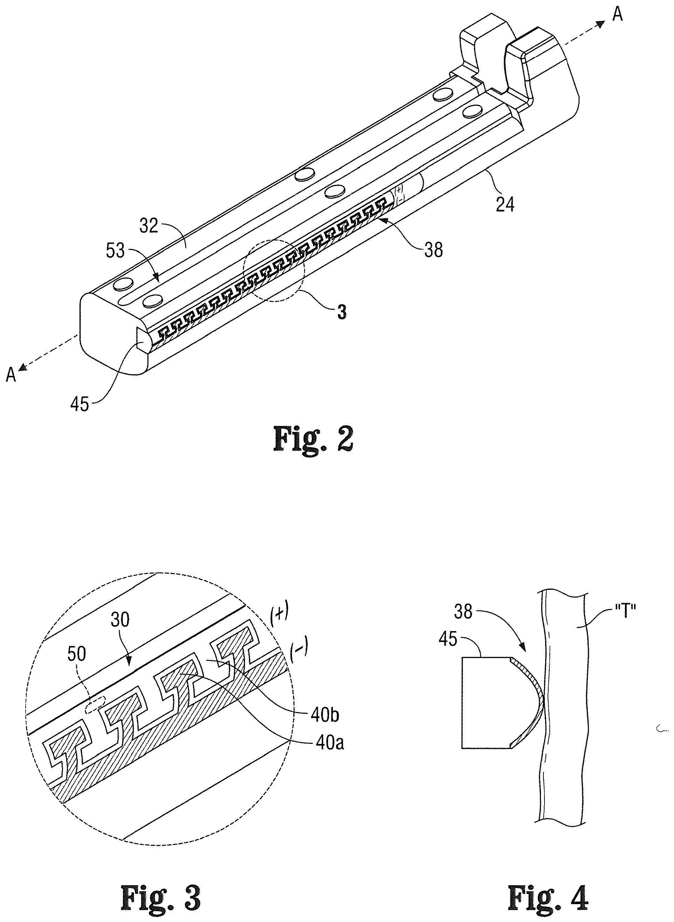

With reference to Fig. 2, in accordance with the instant disclosure one or more second electrode configurations 38 may be disposed on one or both of jaw members 22, 24. In the illustrated embodiment, for example, each jaw member 22, 24 includes second electrode configuration 38 (see Fig. 1 in combination with Fig. 2).

Second electrode configuration 38 is configured to electrosurgically treat tissue in a bipolar mode of operation. Specifically, second electrode configuration 38 includes interlaced second and third electrodes 40a, 40b (interlaced electrodes 40a, 40b) having opposite polarities that are operable in a bipolar mode of operation similar to that of electrodes 30, 32. As defined herein, interlaced means "bind intricately together; interweave." Interlaced electrodes 40a, 40b have a T-shaped pattern (as in the illustrated embodiment). In accordance with the instant disclosure, the interlaced electrodes 40a, 40b are less sensitive to orientation and function more consistently at various approach angles.

Second electrode configuration 38 may extend outwardly from one or both sides of jaw members 22, 24. In Figs. 1 and 2, for example, second electrode configuration 38 is shown extending laterally along jaw member 24 so as to form two separate second electrode configurations 38. In embodiments, second electrode configuration 38 may extend along an entire peripheral edge of jaw members 22, 24 so as to form a single second electrode configuration 38. Accordingly, when tissue is grasped between jaw members 22, 24 and being electrosurgically treated by electrodes 30, 32, a surgeon can roll or tilt jaw members 22, 24 about the grasped tissue such that second electrode configuration 38 can electrosurgically treat tissue, e.g., dissect tissue, at the same time or upon selective subsequent activation. Alternatively, a surgeon can utilize second electrode configuration 38 to electrosurgically treat tissue without tissue being positioned jaw members 22, 24; in this instance, jaw members 22, 24 may be in an open or clamping configuration.

Second electrode configuration 38 may be provided on jaw members 22, 24 via any suitable method or process including but not limited to photo etching process, conductive ink deposition process, laser deposition process, and a stamping process. One or more leads (not explicitly shown) of cable 20 may be utilized to couple second electrode configuration 38 to the generator and/or one or more modules associated therewith, e.g., a microprocessor (not explicitly shown). For example, the leads utilized to couple electrodes 30, 32 to the generator may also be utilized to couple second electrode 30 to the generator. Alternatively, second electrode configuration 38 may have one or more dedicated leads associated therewith to provide electrical continuity between the generator and second electrode configuration 38. In one particular embodiment, for example, each of interlaced electrodes 40a, 40b may have its own dedicated lead operably coupled thereto.

In an embodiment, second electrode configuration 38 may be configured to be active when electrodes 30, 32 on jaw members 22, 24 are active. Thus, in this embodiment, a user may electrosurgically treat tissue grasped between jaw members 22, 24 and tissue outside the grasp of jaw members 22, 24. Alternatively, second electrode configuration 38 may be configured to be inactive when electrodes 30, 32 are active and active when the electrodes 30, 32 are inactive. One or more switches (not explicitly shown) may be employed for this purpose.

In the illustrated embodiment, second electrode configuration 38 extends laterally along jaw members 22, 24 (see Fig. 1 in combination with Fig. 2). Accordingly, a flange 45 may be provided on jaw members 22, 24 and may be configured to extend second electrode 38 outwardly from jaw members 22, 24 and away from electrodes 30, 32. Flange 45 may be integrally formed with jaw members 22, 24 or may be a separate component that is coupled to jaw members 22, 24. In either instance, flange 45 is non-conductive and is utilized to isolate electrodes 30, 32 from second electrode 38 so as to prevent shorts and/or arcing from developing therebetween.

In use, tissue may be grasped by jaw members 22, 24 and electrodes 30, 32 may be active to electrosurgically treat the tissue. As electrodes 30, 32 are electrosurgically treating tissue, second electrode configuration 38 may be active to electrosurgically treat tissue in a manner as described above, e.g., a surgeon can roll or tilt jaw members 22, 24 to treat tissue. Alternatively, second electrode configuration may be active and electrodes 30, 32 may be inactive. In this instance, a surgeon can grasp and electrosurgically treat tissue via second electrode configuration 38. Or, a surgeon can simply move second electrode configuration 38 into contact with tissue "T" (see Fig. 4 for example) for dissection.

The unique configuration of second electrode configuration 38 that includes interlaced electrodes 40a, 40b allows a surgeon to electrosurgically treat tissue without having to worry about an orientation of the electrodes, e.g., especially in the hard to reach areas of a body cavity. The unique configuration of second electrode configuration 38 that includes interlaced electrodes 40a, 40b also allows a surgeon to utilize forceps 2 to perform different electrosurgical procedures, e.g., sealing, dissection.

From the foregoing and with reference to the various figure drawings, those skilled in the art will appreciate that certain modifications can also be made to the present disclosure without departing from the scope of the accompanying claims. For example, in certain instances one or more sensors 50 (shown in phantom in Fig. 3) may be provided adjacent second electrode configuration 38 and utilized to provide feedback information to the microprocessor of the generator.

The various embodiments disclosed herein may also be configured to work with robotic surgical systems and what is commonly referred to as "Telesurgery". Such systems employ various robotic elements to assist the surgeon in the operating theatre and allow remote operation (or partial remote operation) of surgical instrumentation. Various robotic arms, gears, cams, pulleys, electric and mechanical motors may be employed for this purpose and may be designed with a robotic surgical system to assist the surgeon during the course of an operation or treatment. Such robotic systems may include, remotely steerable systems, automatically flexible surgical systems, remotely flexible surgical systems, remotely articulating surgical systems, wireless surgical systems, modular or selectively configurable remotely operated surgical systems.

The robotic surgical systems may be employed with one or more consoles that are next to the operating theater or located in a remote location. In this instance, one team of surgeons or nurses may prep the patient for surgery and configure the robotic surgical system with one or more of the instruments disclosed herein while another surgeon or group of surgeons) remotely control the instruments via the robotic surgical system. As can be appreciated, a highly skilled surgeon may perform multiple operations in multiple locations without leaving his/her remote console which can be both economically advantageous and a benefit to the patient or a series of patients.

The robotic arms of the surgical system are typically coupled to a pair of master handles by a controller. The handles can be moved by the surgeon to produce a corresponding movement of the working ends of any type of surgical instrument (e.g., end effectors, graspers, knifes, scissors) which may complement the use of one or more of the embodiments described herein. The movement of the master handles may be scaled so that the working ends have a corresponding movement that is different, smaller or larger, than the movement performed by the operating hands of the surgeon. The scale factor or gearing ratio may be adjustable so that the operator can control the resolution of the working ends of the surgical instrument(s).

The master handles may include various sensors to provide feedback to the surgeon relating to various tissue parameters or conditions, e.g., tissue resistance due to manipulation, cutting or otherwise treating, pressure by the instrument onto the tissue, tissue temperature, or tissue impedance. As can be appreciated, such sensors provide the surgeon with enhanced tactile feedback simulating actual operating conditions. The master handles may also include a variety of different actuators for delicate tissue manipulation or treatment further enhancing the surgeon's ability to mimic actual operating conditions.

While several embodiments of the disclosure have been shown in the drawings, it is not intended that the disclosure be limited thereto. Those skilled in the art will envision other modifications within the scope of the claims appended hereto.

## Claims

1. An electrosurgical forceps (2), comprising:
a housing (4) having a shaft (14) extending therefrom;
an end effector assembly (12) operably coupled to a distal end (16) of the shaft and including a pair of first and second jaw members (22, 24), at least one of the first and second jaw members movable from an open configuration for positioning tissue therebetween, to a clamping configuration for grasping tissue therebetween, each of the first and second jaw members including a first electrode (30, 32) thereon for electrosurgically treating tissue grasped therebetween; and
second and third electrodes (40a, 40b) disposed on one of the first and second jaw members (22, 24) and separated by an insulator, the second and third electrodes configured to function in a bipolar configuration,
**characterized in that** the second and third electrodes (40a, 40b) on the said at least one of the first and second jaw members (22, 24) are arranged in an interlaced configuration relative to one another, wherein the interlaced configuration is a T-shaped pattern.

2. An electrosurgical forceps according to claim 1, wherein the second and third electrodes (40a, 40b) extend outwardly from one or both sides of the first or second jaw member (22, 24).

3. An electrosurgical forceps according to claim 1 or 2, wherein the second and third electrodes (40a, 40b) are provided on the first or second jaw member (22, 24) via a photo etching process, conductive ink deposition process, laser deposition process, or a stamping process.

4. An electrosurgical forceps according to any preceding claim, wherein the second and third electrodes (40a, 40b) are configured to be active when the first electrodes (30, 32) on the first and second jaw members (22, 24) are active.

5. An electrosurgical forceps according to any one of claims 1 to 3, wherein the second and third electrodes (40a, 40b) are configured to be inactive when the first electrodes (30, 32) on the first and second jaw members (22, 24) are active and to be active when the first electrodes on the first and second jaw members are inactive.

6. An electrosurgical forceps according to any preceding claim, wherein the second and third electrodes (40a, 40b) are provided on both of the first and second jaw members (22, 24).

## Patentansprüche

1. Elektrochirurgische Zange (2), die Folgendes umfasst:
ein Gehäuse (4), das einen Schaft (14) aufweist, der sich davon erstreckt;
eine Endeffektoranordnung (12), die mit einem distalen Ende (16) des Schafts wirkgekoppelt ist und ein Paar eines ersten und eines zweiten Backenelements (22, 24) beinhaltet, wobei das erste und/oder das zweite Backenelement aus einer offenen Konfiguration zum Positionieren von Gewebe dazwischen in eine Klemmkonfiguration zum Greifen von Gewebe dazwischen bewegbar ist, wobei jedes des ersten und des zweiten Backenelements eine erste Elektrode (30, 32) zum elektrochirurgischen Behandeln von Gewebe beinhaltet, das dazwischen gegriffen ist; und
eine zweite und eine dritte Elektrode (40a, 40b), die auf einem des ersten und des zweiten Backenelements (22, 24) angeordnet und durch einen Isolator getrennt sind, wobei die zweite und die dritte Elektrode konfiguriert sind, um in einer bipolaren Konfiguration zu funktionieren,
**dadurch gekennzeichnet, dass** die zweite und die dritte Elektrode (40a, 40b) auf dem ersten und/oder dem zweiten Backenelement (22, 24) in einer verflochtenen Konfiguration relativ zueinander eingerichtet sind, wobei die verflochtene Konfiguration ein T-förmiges Muster ist.

2. Elektrochirurgische Zange nach Anspruch 1, wobei sich die zweite und die dritte Elektrode (40a, 40b) von einer oder beiden Seiten des ersten oder des zweiten Backenelements (22, 24) nach außen erstrecken.

3. Elektrochirurgische Zange nach Anspruch 1 oder 2, wobei die zweite und die dritte Elektrode (40a, 40b) auf dem ersten oder dem zweiten Backenelement (22, 24) über einen Fotoätzvorgang, einen leitfähigen Tintenabscheidungsvorgang, einen Laserabscheidungsvorgang oder einen Stanzvorgang bereitgestellt sind.

4. Elektrochirurgische Zange nach einem der vorhergehenden Ansprüche, wobei die zweite und die dritte Elektrode (40a, 40b) konfiguriert sind, um aktiv zu sein, wenn die ersten Elektroden (30, 32) an dem ersten und dem zweiten Backenelement (22, 24) aktiv sind.

5. Elektrochirurgische Zange nach einem der Ansprüche 1 bis 3, wobei die zweite und die dritte Elektrode (40a, 40b) konfiguriert sind, um inaktiv zu sein, wenn die ersten Elektroden (30, 32) an dem ersten und dem zweiten Backenelement (22, 24) aktiv sind und aktiv sein sollen, wenn die ersten Elektroden auf dem ersten und dem zweiten Backenelement inaktiv sind.

6. Elektrochirurgische Zange nach einem der vorhergehenden Ansprüche, wobei die zweite und die dritte Elektrode (40a, 40b) sowohl an dem ersten als auch an dem zweiten Backenelement (22, 24) bereitgestellt sind.

## Revendications

1. Pince électrochirurgicale (2), comprenant :
un logement (4) ayant un arbre (14) s'étendant à partir de celui-ci ;
un ensemble d'organe effecteur (12) accouplé de manière fonctionnelle à une extrémité distale (16) de l'arbre et comportant une paire de premier et second éléments de mâchoire (22, 24), le premier et/ou le second élément de mâchoire déplaçable d'une configuration ouverte pour positionner le tissu entre eux, à une configuration de serrage pour saisir le tissu entre eux, chacun des premier et second éléments de mâchoire comportant une première électrode (30, 32) sur celui-ci pour traiter de manière électrochirurgicale le tissu saisi entre eux ; et
des deuxième et troisième électrodes (40a, 40b) disposées sur l'un des premier et second éléments de mâchoire (22, 24) et séparées par un isolant, les deuxième et troisième électrodes étant conçues pour fonctionner dans une configuration bipolaire,
**caractérisé en ce que** les deuxième et troisième électrodes (40a, 40b) sur ledit un premier et/ou un second élément de mâchoire (22, 24) sont agencées dans une configuration entrelacée l'une par rapport à l'autre, la configuration entrelacée étant un motif en forme de T.

2. Pince électrochirurgicale selon la revendication 1, dans laquelle les deuxième et troisième électrodes (40a, 40b) s'étendent vers l'extérieur à partir d'un côté ou des deux côtés du premier ou du second élément de mâchoire (22, 24).

3. Pince électrochirurgicale selon la revendication 1 ou 2, dans laquelle les deuxième et troisième électrodes (40a, 40b) sont prévues sur le premier ou le second élément de mâchoire (22, 24) par l'intermédiaire d'un processus de photogravure, d'un processus de dépôt d'encre conductrice, d'un processus de dépôt laser ou d'un processus d'estampage.

4. Pince électrochirurgicale selon l'une quelconque des revendications précédentes, dans laquelle les deuxième et troisième électrodes (40a, 40b) sont conçues pour être actives lorsque les premières électrodes (30, 32) sur les premier et second éléments de mâchoire (22, 24) sont actives.

5. Pince électrochirurgicale selon l'une quelconque des revendications 1 à 3, dans laquelle les deuxième et troisième électrodes (40a, 40b) sont conçues pour être inactives lorsque les premières électrodes (30, 32) sur les premier et second éléments de mâchoire (22, 24) sont actives et pour être actives lorsque les premières électrodes sur les premier et second éléments de mâchoire sont inactives.

6. Pince électrochirurgicale selon l'une quelconque des revendications précédentes, dans laquelle les deuxième et troisième électrodes (40a, 40b) sont prévues à la fois sur les premier et second éléments de mâchoire (22, 24).
